# EUROPÄISCHE PATENTANMELDUNG

(11) **EP 4 289 933 A1**
(43) Veröffentlichungstag der Anmeldung: **13.12.2023**
(21) Anmeldenummer: 22177581.0
(22) Anmeldetag: 07.06.2022
(51) Int. Cl.: C12M 3/06, C12M 1/00

(54) **ZELLKULTURTRÄGER**

(71) Anmelder: ibidi GmbH, 82166 Gräfelfing (DE); Technische Universität Darmstadt, 64289 Darmstadt (DE)
(72) Erfinder: von Guttenberg, Zeno, 82194 Gröbenzell (DE); Fritschen, Anna, 64289 Darmstadt (DE); Blaeser, Andreas, 53840 Troisdorf (DE)
(74) Vertreter: Grünecker Patent- und Rechtsanwälte PartG mbB

(57) **Zusammenfassung**

Die Erfindung betrifft einen Zellkulturträger umfassend einen ersten Kanal und ein Reservoir, wobei der erste Kanal und das Reservoir im Inneren des Zellkulturträgers ausgebildet sind, wobei der erste Kanal eine Einlassöffnung in einer Außenseite des Zellkulturträgers und eine Auslassöffnung in der Außenseite des Zellkulturträgers aufweist, wobei das Reservoir mindestens zwei nebeneinander angeordnete Öffnungen aufweist, wobei der erste Kanal durch die mindestens zwei Öffnungen mit dem Reservoir verbunden ist, und wobei das Reservoir durch keinen weiteren Kanal mit der Außenseite des Zellkulturträgers verbunden ist.

## Beschreibung

Die vorliegende Erfindung betrifft einen Zellkulturträger, einen Zellkulturträgerbausatz, sowie ein Verfahren zum Einbringen von Zellen oder Zellaggregaten in einen derartigen Zellkulturträger.

Zellkulturträger werden unter anderem eingesetzt, um das Wachstum und das Verhalten lebender Zellen unter kontrollierten Umwelteinflüssen zu simulieren und zu untersuchen. Dazu müssen die Zellen in einem definierten Volumen, beispielsweise einem Hohlraum im Zellkulturträger, festgehalten werden, um sie dort beobachten zu können, beispielsweise via Mikroskopie. Während der Kultivierung und Untersuchung werden die Zellen typischerweise mittels eines Zellmediums mit Nährstoffen versorgt. Insbesondere beim Austausch dieses Zellmediums ist es von Bedeutung, dass die Zellen weder einer direkten Strömung ausgesetzt sind, noch aus dem genannten Volumen gespült werden.

Grundsätzlich unterscheidet man zwischen zwei verschiedenen Zelltypen, adhärenten und nicht adhärenten Zellen. Adhärente Zellen können an einer Oberfläche oder einem Substrat adhärieren, wobei die Beschaffenheit der Oberfläche einen Einfluss auf die Adhäsionsfähigkeit der Zellen hat. Adhärente Zellen werden nicht von der Oberfläche abgelöst, wenn das Zellmedium ausgetauscht wird. Können Zellen nicht an einer Oberfläche adhärieren, bilden sie durch Adhäsion aneinander Zellaggregate, sogenannte Sphäroide. Diese sind entsprechend nicht mit der Oberfläche verbunden und werden durch einen Strom aus einem Zellmedium fortbewegt.

Das physiologische Verhalten der Zellen hängt ebenfalls davon ab, ob sie adhäriert vorliegen oder im Zellmedium suspendiert sind. So können bestimmte Zellen, beispielsweise Immunzellen, durch den Kontakt mit einer Oberfläche ihre Eigenschaften verändern. Möchte man Zellen über längere Zeiträume beobachten, müssen nicht adhärente Zellen folglich an einem Ort festgehalten werden, ohne dass sie bei einem Austausch des Zellmediums mitgeführt werden. Bei Untersuchungen, die an Sphäroiden durchgeführt werden sollen, muss gleichzeitig verhindert werden, dass adhärente Zellen an einer Oberfläche adhärieren, da das sich das Zellaggregat sonst entsprechend zu einer Zellschicht umstrukturiert. Weiterhin kommen die wenigsten Zellen in natürlichen Organstrukturen in direkten Kontakt mit einem Blutstrom und sind somit keinem Scherstress ausgesetzt. Sie erhalten in der Regel ihre Nährstoffe mittels Diffusion durch das intrazelluläre Gewebe. Entsprechend dürfen diese Zellen in einem Zellkulturträger, um physiologische Bedingungen zu erzeugen, keiner direkten Strömung des Zellmediums ausgesetzt sein, also nicht perfundiert werden.

Aus WO 2016/076795 A1 ist eine mikrofluidische Plattform zur Untersuchung zellbasierter Wechselwirkungen bekannt, umfassend drei nebeneinander angeordnete mikrofluidische Kanäle. Zwischen dem mittleren Kanal und den jeweils außen angeordneten Kanälen findet ein Flüssigkeitsaustausch statt, sodass ein Zellmedium zwischen den Kanälen ausgetauscht werden kann. Der mittlere Kanal ist mit einem Hydrogel oder einer extrazellulären Matrix gefüllt, worin sich Zellen befinden. Dabei umfasst der mittlere Kanal Bereiche, die keine Verbindung mit den anderen Kanälen aufweisen, sodass in diesen Bereichen kein Kontakt mit dem Zellmedium stattfindet. Dies betrifft insbesondere die Abschnitte zwischen der Kanalmündung in der Außenseite der Plattform und dem Abschnitt, in dem eine Verbindung mit den anderen beiden Kanälen hergestellt ist. In besagten Bereichen befindliche Zellen können nicht oder nicht ausreichend mit Nährstoffen versorgt werden und dadurch absterben. Aus diesem Grund gibt es in diesem Kanal unbenutzte Bereiche, in denen keine biologischen Untersuchungen durchgeführt werden können. Die Bereiche können daher Totbereiche genannt werden.

Im Lichte dieser Nachteile ist es eine Aufgabe der vorliegenden Erfindung, einen Zellkulturträger, sowie einen zugehörigen Zellkulturträgerbausatz und ein Verfahren zum Einbringen von Zellen oder Zellaggregaten in einem derartigen Zellkulturträger bereitzustellen, in dem sich keine Totbereiche ausbilden oder die Ausbildung von Totbereichen unterdrückt ist, sodass ein für die Untersuchung von Zellen verfügbares Volumen vollständig genutzt werden kann.

Diese Aufgabe wird durch den Zellkulturträger nach Anspruch 1, den Zellkulturträgerbausatz nach Anspruch 11 und das Verfahren nach Anspruch 13 gelöst. Besonders vorteilhafte Ausbildungen finden sich in den zugehörigen Unteransprüchen.

Erfindungsgemäß wird ein Zellkulturträger bereitgestellt, umfassend einen ersten Kanal und ein Reservoir, wobei der erste Kanal und das Reservoir im Inneren des Zellkulturträgers ausgebildet sind, wobei der erste Kanal eine Einlassöffnung in einer Außenseite des Zellkulturträgers und eine Auslassöffnung in der Außenseite des Zellkulturträgers aufweist, wobei das Reservoir mindestens zwei nebeneinander angeordnete Öffnungen aufweist, wobei der erste Kanal durch die mindestens zwei Öffnungen mit dem Reservoir verbunden ist, und wobei das Reservoir durch keinen weiteren Kanal mit der Außenseite des Zellkulturträgers verbunden ist.

Durch die Öffnungen zwischen Reservoir und Kanal kann ein durch den Kanal strömendes Zellmedium in das Reservoir gelangen und dort verteilt werden. Das Reservoir ist lediglich über den Kanal mit einer Außenseite des Zellkulturträgers verbunden, weil es keinen Kanal gibt, der direkt in das Reservoir mündet. Daher sind Zellen oder Zellaggregate, die sich im Reservoir befinden, auf den Bereich des Reservoirs räumlich eingeschränkt und die oben genannten Abschnitte zwischen einer Kanalmündung und dem Reservoir kommen nicht vor. Insbesondere sind die Zellen auf einen Bereich eingeschränkt, der mit dem Zellmedium versorgt werden kann, sodass das gesamte Reservoir für die Untersuchung von Zellen genutzt werden kann.

Als Außenseite des Zellkulturträgers werden hierbei dessen gesamte nach außen weisende Oberflächen bezeichnet. Insbesondere sind die Innenflächen des Kanals und des Reservoirs hiervon nicht umfasst.

Ein Kanal ist beispielsweise eine im Zellkulturträger ausgebildete Röhre. Ein Kanal umfasst dabei zwei Öffnungen oder Mündungen, die in diesem Fall die Einlassöffnung und die Auslassöffnung sind. Beide Öffnungen sind in einer Außenseite des Zellkulturträgers angeordnet und können dabei beispielsweise als Loch in der Außenseite ausgebildet sein.

Der Kanal kann einen Durchmesser, beziehungsweise eine Breite eines Querschnitts, aufweisen, die kleiner ist als die Länge des Kanals. Die Länge kann hierbei die Distanz zwischen der Einlassöffnung und der Auslassöffnung des Kanals sein, wobei man zur Bestimmung der Länge eines Kanals diesen von seiner Einlassöffnung zu seiner Auslassöffnung durchläuft. Das Verhältnis zwischen der Breite eines Querschnitts und der Länge eines Kanals kann dabei einen Wert von 0,2 oder weniger, insbesondere 0,1 oder weniger, insbesondere 0,05 oder weniger aufweisen. Ein Reservoir hingegen kann einen Durchmesser oder eine Breite aufweisen, die größer als die Höhe des Reservoirs ist. Die Höhe des Reservoirs ist der vertikale Abstand zwischen begrenzenden Flächen des Reservoirs. Das Verhältnis zwischen der Breite des Reservoirs und der Höhe des Reservoirs kann einen Wert von 2 oder mehr, insbesondere 5 oder mehr, insbesondere 10 oder mehr aufweisen.

Da ein Medium den Kanal zwischen Einlassöffnung und Auslassöffnung durchströmt, liegen die Öffnungen, durch die der Kanal mit dem Reservoir verbunden ist, nicht in Strömungsrichtung eines durch den Kanal strömenden Mediums. Die Öffnungen können insbesondere seitlich im Kanal angeordnet sein. Das Medium strömt also im Wesentlichen an den Öffnungen vorbei und es findet ein Austausch mit dem Inneren des Reservoirs lediglich durch Diffusion infolge eines Konzentrationsgradienten zwischen Kanal und Reservoir statt und nicht durch einen direkten Fluss. Insbesondere kann der Kanal derart angeordnet sein, dass er das Reservoir tangiert oder entlang eines bestimmten Abschnitts parallel zur Seitenwand des Reservoirs verläuft.

Für das Reservoir als Hohlraum im Zellkulturträger sind unterschiedliche Ausführungsformen, insbesondere geometrische Formen möglich. So kann es beispielsweise eine zylindrische Form aufweisen. Die Öffnungen können in der Mantelfläche des Zylinders angeordnet sein. Statt eines Zylinders kann das Reservoir auch die Form eines Prismas aufweisen. In diesem Fall lässt sich auch eine Seitenfläche des Reservoirs definieren, analog zur Mantelfläche eines Zylinders. Insbesondere können die Öffnungen in der Seitenfläche des Reservoirs angeordnet sein. Darüber hinaus ist es möglich, dass das Reservoir die Form einer Kugel oder eines Rotationsellipsoids aufweist.

Der Zellkulturträger kann genau einen Kanal umfassen.

Das Reservoir des Zellkulturträgers kann mit genau einem Kanal verbunden sein.

Aus diesen Formulierungen folgt, dass das Reservoir über keinen weiteren Kanal mit der Außenseite des Zellkulturträgers verbunden ist.

Das Reservoir kann weiterhin eine kreisförmige, elliptische oder rechteckige Querschnittsfläche aufweisen. Ein Verhältnis zweier Hauptausdehnungen des Querschnitts des Reservoirs kann im Bereich zwischen 0,5 und 2 liegen, wobei der Querschnitt durch eine Ebene definiert ist, die parallel zu einer Grundfläche des Zellkulturträgers liegt.

Eine kreisförmige oder elliptische Querschnittsform des Reservoirs erlaubt eine zuverlässige Verteilung eines Zellkulturmediums (im Folgenden auch als Zellmedium bezeichnet) im Reservoir, das durch Diffusion durch die Öffnungen in das Reservoir transportiert wird. Dies trägt zusätzlich dazu bei, dass Totbereiche, in denen ein Austausch des Zellmediums nur auf langen Zeitskalen stattfindet, vermieden werden können. Weiterhin bietet eine runde oder elliptische Form den Vorteil, dass das Verhältnis der Oberfläche zum Volumen besonders gering ist. Eine geringere Oberfläche verringert die Wahrscheinlichkeit, dass Zellen an den Wänden des Reservoirs adhärieren.

Eine rechteckige Querschnittsform erlaubt eine besonders einfache Geometrie der Anordnung von Reservoir und Kanal. Insbesondere kann der Kanal geradlinig und parallel zu einer Rechteckseite verlaufen und entlang der Rechteckseite eine Mehrzahl von Öffnungen, insbesondere mehr als zwei Öffnungen, aufweisen. Mehrere Öffnungen erlauben einen zuverlässigeren und schnelleren Austausch eines Mediums zwischen dem Kanal und dem Reservoir.

Durch die Wahl der Hauptausdehnungen des Querschnitts kann ein kleineres Volumen des Reservoirs als im Stand der Technik ermöglicht werden, während der Austausch des Zellmediums effizient stattfindet. Ein geringes Reservoirvolumen hat hierbei den Vorteil, dass eine geringere Anzahl an Zellen und eine geringere Menge an zusätzlichen Materialien wie Hydrogelen für die Untersuchungen eingesetzt werden müssen. Auf diese Weise können die Kosten für experimentelle Untersuchungen gesenkt werden. Abhängig von der Form des Reservoirs und der Anordnung und der Anzahl der Öffnungen kann auch ein Austausch des Zellmediums in einem geringen Volumen innerhalb eines kürzeren Zeitraums stattfinden und die Versorgung von Zellen im Reservoir mit Nährstoffen verbessert werden.

Unter einer Hauptausdehnung kann dabei eine größte oder eine charakteristische Länge der geometrischen Querschnittsform verstanden werden. Im Fall eines elliptischen Querschnitts entsprechen die beiden Hauptausdehnungen der großen und der kleinen Halbachse der Ellipse. Das Reservoir kann auch einen rechteckigen Querschnitt aufweisen. In diesem Fall wären die Hauptausdehnungen die Seitenlängen des Rechtecks. Die Form des Querschnitts ist allerdings nicht auf die genannten Beispiele beschränkt und kann beliebig ausgeführt sein, da sich für jede geometrische Form Hauptausdehnungen definieren lassen.

Der Zellkulturträger kann eine Grundfläche aufweisen, die insbesondere plan ausgebildet sein kann. Im bestimmungsgemäßen Gebrauch bildet die Grundfläche die Unterseite des Zellkulturträgers. Sofern nicht anders spezifiziert, sind im Folgenden alle Angaben, die sich direkt oder indirekt auf den Zellkulturträger und die räumliche Anordnung dessen Komponenten beziehen, also beispielsweise "oberhalb", "unterhalb", "oben", oder "unten" mit Bezug auf die Grundfläche des Zellkulturträgers zu verstehen.

Der Zellkulturträger kann einen zweiten Kanal umfassen, wobei der zweite Kanal durch mindestens zwei weitere Öffnungen mit dem Reservoir verbunden ist.

Die Ausbildung eines zweiten Kanals im Zellkulturträger erhöht die Flexibilität und die Einsatzmöglichkeiten desselben. Beispielsweise kann der zweite Kanal von einem anderen Medium durchströmt werden als der erste Kanal. Durch die Verbindung des Reservoirs mit einem zweiten Kanal erhöht sich die Austauschrate eines Mediums zwischen den Kanälen und dem Reservoir, sodass der Austausch des Zellmediums zwischen den Kanälen und dem Reservoir innerhalb eines kürzeren Zeitraums ablaufen kann als im Fall nur eines einzigen Kanals. Dadurch kann beispielsweise eine bessere Versorgung von Zellen im Reservoir mit Nährstoffen erreicht werden.

Der Zellkulturträger kann genau zwei Kanäle, nämlich den ersten Kanal und den zweiten Kanal, umfassen.

Das Reservoir des Zellkulturträgers kann nur mit dem ersten Kanal und dem zweiten Kanal verbunden sein.

Für die genannten Spezialfälle, dass der Kanal genau einen oder genau zwei Kanäle umfasst, folgt ebenfalls, dass das Reservoir durch keinen weiteren Kanal als dem ersten Kanal und dem zweiten Kanal mit der Außenseite des Zellkulturträgers verbunden ist. Mit anderen Worten, der Zellkulturträger umfasst keinen Kanal, der im Reservoir endet oder das Reservoir ist nur mit Kanälen verbunden, die genau zwei Öffnungen in der Außenseite des Zellkulturträgers, insbesondere eine Einlassöffnung und eine Auslassöffnung, aufweisen.

Die Öffnungen zwischen dem Reservoir und dem ersten Kanal und dem Reservoir und dem zweiten Kanal können an gegenüberliegenden Stellen im Reservoir angeordnet sein.

Die Ausbildung von Öffnungen, die die Kanäle mit dem Reservoir verbinden, an gegenüberliegenden Stellen des Reservoirs, ermöglichen einen verbesserten Flüssigkeitsaustausch zwischen den Kanälen und dem Reservoir. Weiterhin wird durch eine größere Anzahl an Öffnungen auch die Möglichkeit verringert, dass sich im Reservoir Totbereiche ausbilden können, in denen der Austausch des Zellmediums zwischen den Kanälen und dem Reservoir nicht oder nur auf langen Zeitskalen stattfindet.

Der Begriff "gegenüberliegend" bedeutet in diesem Zusammenhang, dass die beiden Stellen in jeweils unterschiedlichen Regionen des Reservoirs angeordnet sind. Beispielsweise können die Regionen auf unterschiedlichen Seiten einer Mittelachse des Reservoirs angeordnet sein. Im Fall eines rechteckigen Reservoirquerschnitts kann auf jeder der beiden parallel verlaufenden Rechteckseiten eine der besagten Stellen angeordnet sein. Für einen kreisförmigen Querschnitt können zwei Halbkreise definiert werden, wobei die beiden Stellen in unterschiedlichen Halbkreisen angeordnet sind. Eine analoge Definition kann auch auf eine Ellipse angewendet werden. In einem Spezialfall können die Stellen, an denen die Öffnungen ausgebildet sind, durch eine gedachte Linie geradlinig verbunden werden können, wobei die Linie durch den geometrischen Mittelpunkt des Reservoirs verläuft.

Ein Abstand zwischen zwei der mindestens zwei benachbarten Öffnungen kann größer als der halbe Wert einer Breite der Öffnungen sein. Der Abstand zwischen zwei der mindestens zwei benachbarten Öffnungen kann kleiner als der fünffache Wert der Breite der Öffnungen sein.

Auf die beschriebene Art und Weise nahe beieinander angeordnete Öffnungen können eine größere Menge eines Mediums in einem definierten Bereich im Reservoir bereitstellen. Beispielsweise können so Bereiche mit einer höheren Nährstoffkonzentration erreicht werden, wenn ein Zellmedium durch den Kanal strömt, was die Zellen in den bezüglichen Bereichen im Reservoir begünstigen kann.

Das Reservoir kann teilweise oder vollständig mit einer zellabweisenden Schicht beschichtet sein.

Durch eine derartige zellabweisende Schicht kann erreicht werden, dass Zellen im Reservoir nicht an einer Innenwand, beispielsweise der Seitenwand, adhärieren können. Wie beschrieben kann eine Adhäsion von Zellen an der Innenwand des Reservoirs deren Eigenschaften verändern, was durch eine zellabweisende Schicht verhindert werden kann.

In einem einfachen Fall kann eine zellabweisende Schicht bereits durch einen unbehandelten Kunststoff gebildet werden, weil Zellen typischerweise auf hydrophoben Oberflächen schlecht adhärieren. Für die zellabweisende Schicht kommen aber auch Beschichtungen aus Polymeren, Proteinen und Lipiden infrage, die auf eine Innenwand des Reservoirs aufgetragen sein können. Die Innenwand des Reservoirs umfasst hierbei alle Flächen des Reservoirs. Es kann die gesamte Innenfläche mit der zellabweisenden Schicht beschichtet sein, es können aber auch nur bestimmte Teile oder Abschnitte besagter Innenfläche mit der zellabweisenden Schicht beschichtet sein.

Der Zellkulturträger kann ein Deckelement und ein Bodenelement umfassen, wobei auf der dem Bodenelement zugewandten Seite des Deckelements ein Graben und eine Vertiefung ausgebildet sind, wobei das Deckelement und das Bodenelement flächig miteinander verbunden sind, sodass der Graben und die Vertiefung durch das Bodenelement abgedeckt sind, und wobei durch die Abdeckung des Grabens der Kanal und durch die Abdeckung der Vertiefung das Reservoir gebildet werden.

Die Verwendung eines Bodenelements vereinfacht die Herstellung des Zellkulturträgers. Beispielsweise kann das Deckelement mittels Spritzguss hergestellt werden, was eine einfache, präzise und kostengünstige Fertigungsmethode darstellt. Während mit Spritzguss keine Hohlräume in einer zu fertigenden Komponente ausgebildet werden können, stellt die Ausbildung einer Vertiefung und/oder eines Grabens an einer Oberfläche eine einfache Alternative dar, um im Zusammenspiel mit einem Bodenelement, um die Vertiefung und den Graben abzudecken, einen äquivalenten Zellkulturträger zu erhalten. Ein weiterer Vorteil der Aufteilung des Zellkulturträgers in ein Deckelement und ein Bodenelement zeigt sich im Zusammenhang mit dessen Verwendung. In die Vertiefung können direkt zu untersuchende Zellen eingebracht werden, bevor das Reservoir durch die Verschließung mit dem Bodenelement gebildet wird.

Der Graben kann sich auch an einen als Röhre ausgebildeten Kanal innerhalb des Deckelements anschließen, sodass der gesamte Kanal durch die Röhre in Verbindung mit dem durch das Bodenelement abgedeckten Graben gebildet wird. Durch die Abdeckung des Grabens mit dem Bodenelement wird schließlich der vollständige Kanal im Zellkulturträger gebildet. Unter einer Abdeckung wird in diesem Zusammenhang insbesondere eine flüssigkeitsdichte Verschließung des Grabens durch das Bodenelement verstanden. Dadurch kann ein Medium, insbesondere eine Flüssigkeit, durch den auf diese Weise gebildeten Kanal strömen, ohne dass es an anderen Stellen als der Einlassöffnung und der Auslassöffnung austreten kann.

Auf die gleiche Weise wie der Graben wird auch die im Deckelement ausgebildete Vertiefung durch das Bodenelement abgedeckt. Dadurch wird das Reservoir gebildet, das einen Hohlraum innerhalb des Zellkulturträgers darstellt.

Diese Zusammenstellung eines Zellkulturträgers mit einem Deckelement und einem Bodenelement ist mit den zuvor beschriebenen Eigenschaften des Zellkulturträgers kombinierbar. Beispielweise kann der Zellkulturträger mehr als einen Kanal umfassen, wobei entsprechend mehr als ein Graben in der Seite des Deckelements ausgebildet sein kann. Insbesondere kann für jeden Kanal des Zellkulturträgers ein Graben in der Seite des Deckelements ausgebildet sein, es können aber auch weniger Gräben vorhanden sein, als es Kanäle im Zellkulturträger gibt. Auch die beschriebenen Formen des Reservoirs, soweit mit dieser Ausführung des Zellkulturträgers vereinbar, können auf den Zellkulturträger, umfassend ein Bodenelement und ein Deckelement, zutreffen.

Der beschriebene Zellkulturträger kann weiterhin einen Klebefilm umfassen, der zwischen dem Deckelement und dem Bodenelement angeordnet ist, sodass das Deckelement und das Bodenelement flächig miteinander verbunden und verklebt sind.

Es gibt mehrere Möglichkeiten, das Deckelement mit dem Bodenelement zu verbinden. Dabei stellt die Verwendung eines Klebefilms eine besonders einfache, anwenderfreundliche und kostengünstige Option dar, wobei allerdings gleichzeitig eine stabile und anhaltende Verbindung des Deckelements mit dem Bodenelement erreicht werden. In dieser Beschreibung werden nachfolgend mehrere Möglichkeiten für die Ausbildung des Klebefilms vorgestellt.

Der Klebefilm kann an der Seite der Oberfläche des Deckelements angeordnet sein, in der auch die Vertiefung und der Graben ausgebildet sind. Diese Seite ist dem Bodenelement zugewandt. Der Klebefilm bedeckt insbesondere nicht die Vertiefung. Insbesondere ist es auch möglich, dass der Klebefilm weder die Vertiefung noch den Kanal bedeckt. Der Klebefilm kann die gesamte verbleibende Fläche bedecken, kann aber auch nur in einzelnen Abschnitten aufgebracht sein, solange dadurch erreicht wird, dass das Deckelement und das Bodenelement stabil miteinander verklebt sind. Der Klebefilm kann eine Dicke zwischen 50 µm und 500 µm, insbesondere zwischen 50 µm und 150 µm aufweisen.

Der Zellkulturträger kann aus dem Deckelement, dem Bodenelement und dem Klebefilm bestehen, also keine weiteren Komponenten umfassen.

Zwei der mindestens zwei nebeneinander angeordneten Öffnungen des ersten Kanals können durch eine Säule getrennt sein, wobei die Säule im Deckelement ausgebildet ist und das Bodenelement berührt, und wobei die dem Deckelement zugewandte Seite des Bodenelements plan ausgebildet ist.

Da die Säule das Bodenelement berührt, entstehen keine Spalte oder anderweitige Zwischenräume zwischen der Säule und dem Bodenelement, durch die Zellen aus dem Reservoir entweichen können. Es gibt dadurch also eine zusätzliche Sicherheit, dass die Zellen innerhalb des Reservoirs eingeschlossen sind und darin festgehalten werden können.

Es sei angemerkt, dass die Säule nicht mit dem Klebefilm bedeckt sein kann, da sie das Bodenelement berührt und damit in Kontakt steht.

Falls der Zellkulturträger einen zweiten Kanal umfasst, können auch zwei der mindestens zwei nebeneinander angeordneten Öffnungen des zweiten Kanals durch eine Säule getrennt sein.

In einer bestimmungsgemäßen Verwendung des Zellkulturträgers kann Mikroskopie am Zellkulturträger durch das Bodenelement durchgeführt werden. Hierauf wird nachfolgend noch genauer eingegangen. In diesem Fall ist eine plan ausgebildete Seite des Bodenelements für optische Untersuchungen von Vorteil, da sie optische Imperfektionen gegenüber einer konturierten Seite unterdrückt. Insbesondere kann zudem die dem Deckelement abgewandte Seite des Bodenelements plan ausgebildet sein, diese beiden Seiten können also plan und parallel sein.

Entlang ihrer Längsachse kann die Säule einen kreisförmigen Querschnitt aufweisen, was im Zusammenhang mit Spritzguss als Fertigungstechnik besonders einfach und präzise herzustellen ist. Der Durchmesser der Säulen kann zwischen 20 µm und 500 µm betragen. Allerdings kann der Querschnitt auch eine andere Form aufweisen, ohne dass die durch den vorliegenden Zellkulturträger gelöste technische Aufgabe dadurch beeinträchtigt wäre. Beispielsweise kommt auch ein elliptischer oder rechteckiger Querschnitt in Betracht. Die genannte Größenskala für den Querschnitt kann auch auf andere Querschnittsformen entsprechend angewendet werden.

Die Höhe der Säule kann größer sein als die Höhe der Vertiefung im Deckelement. Dabei ist die Höhe der Vertiefung den vertikalen Abstand zwischen dem Boden der Vertiefung und der Seite der Oberfläche des Deckelements, in der die Vertiefung ausgebildet ist. Die Säule kann also über die Oberfläche des Deckelements hinausragen. Insbesondere kann die Differenz der Höhe der Säule und der Höhe der Vertiefung der Dicke des Klebefilms entsprechen. Dadurch kann sichergestellt werden, dass das Deckelement und das Bodenelement miteinander verbunden werden, sich also berühren, ohne unerwünschte Spalte oder Lücken zwischen den beiden Elementen zu erzeugen, durch die Zellen aus dem Reservoir entweichen könnten. Dies bedeutet gleichzeitig auch, dass die Säule nicht mit dem Klebefilm bedeckt sein kann.

Das Reservoir kann mehr als zwei benachbarte Öffnungen aufweisen. Da eine Säule zwischen zwei benachbarten Öffnungen angeordnet sein kann, um diese zu trennen, kann in diesem Fall auch mehr als eine Säule vorhanden sein. Wenn N die Zahl an Öffnungen bezeichnet, so können entsprechend N-1 Säulen ausgebildet sein, die diese N Öffnungen voneinander trennen. Von diesen N-1 Säulen kann eine Säule das Bodenelement berühren, aber es können auch mehrere oder alle Säulen das Bodenelement berühren, beziehungsweise einen direkten Kontakt mit dem Bodenelement herstellen.

Durch eine Mehrzahl von Öffnungen wird der Medienaustausch zwischen dem Kanal, beziehungsweise den Kanälen, und dem Reservoir verbessert, während gleichzeitig kein erhöhtes Risiko besteht, dass die Zellen aus dem Reservoir entweichen können.

Weiterhin können die nebeneinander in der Seitenwand angeordneten Öffnungen durch eine Mehrzahl von Säulen getrennt sein, wobei die Mehrzahl von Säulen in Form eines Gitters angeordnet sind. Das Gitter kann insbesondere ein zweidimensionales Gitter sein. In diesem Fall definiert eine Reihe von nebeneinander angeordneten Säulen, die dem Reservoir an nächsten sind, die Öffnungen des Reservoirs. Die Zahl an Öffnungen des Reservoirs wird demnach durch die Anordnung der Säulen in einem zweidimensionalen Gitter nicht verändert. Weitere Säulen der Mehrzahl von Säulen befinden sich folglich weiter entfernt vom Reservoir. Unter einem Gitter wird hierin eine regelmäßige Anordnung der Säulen verstanden, wobei die Positionen der Säulen translationssymmetrisch angeordnet sein. Beispielsweise können die Säulen in Form eines quadratischen, dreieckigen oder hexagonalen Gitters angeordnet sein. Weiterhin können manche der weiter vom Reservoir entfernten Säulen im Kanal angeordnet sein.

Die Öffnungen können so eng ausgebildet sein, dass Zellen oder Zellaggregate im Reservoir dieses nicht verlassen und somit nicht in den Kanal gelangen können. Dies kann beispielweise dadurch bewerkstelligt werden, dass der Abstand zwischen den Säulen und damit die Breite der Öffnungen kleiner ist als die typische Größenskala der im Reservoir befindlichen Zellen oder Zellaggregate. Eine Mehrzahl von Säulen, die in einem Gitter angeordnet sind, reduzieren die Möglichkeit weiter, weil neben den Öffnungen noch weitere Engstellen ausgebildet sind, wodurch die Zellen hindurchtreten müssten.

Das Reservoir des Zellkulturträgers kann mit einem Gel befüllt sein, das Zellen oder Zellaggregate enthält.

Durch die Bereitstellung eines mit Gel befüllten Zellkulturträgers kann ein Anwender des Zellkulturträgers diesen direkt für die vorgesehene Verwendung einsetzen. Beispielsweise kann der Zellkulturträger dazu genutzt werden, nicht adhärente Zellen im Reservoir festzuhalten und zu untersuchen, beispielsweise via Mikroskopie. Durch den Kanal kann ein Zellmedium strömen, das durch Diffusion durch die Öffnungen in das Reservoir gelangt und das Zellmedium entsprechend ausgetauscht wird, ohne dass die Zellen einer direkten Strömung ausgesetzt sind.

Dadurch werden die Zellen oder Zellaggregate keinem Scherstress ausgesetzt. Dies hat den Vorteil, einen physiologischen Zustand zu erreichen, da im menschlichen Körper nur wenige Zelltypen wie beispielsweise Endothelzellen einer Strömung ausgesetzt sind, während die meisten Zelltypen Nährstoffe mittels Diffusion aufnehmen. Diese Situation besitzt eine hohe physiologische Relevanz, kommt also in der Natur vorkommenden Szenarien besonders nahe.

Das Gel kann insbesondere ein Hydrogel sein, das einem Gel aus vernetzten Polymeren entspricht, welches Wasser binden kann. Dieses kann die Materialien GelMA, Alginat, Kollagen oder Fibrin umfassen oder daraus bestehen. Bei der Wahl des Materials gilt es zu beachten, dass es entsprechend vernetzt können werden muss, um ein Gel zu bilden. Kollagen kann beispielsweise durch thermischen Einfluss vernetzt werden. Dem gegenüber kann Alginat chemisch vernetzt werden, während bei GelMA die Vernetzung durch UV-Strahlung erzielt werden kann. Im Fall von Fibrin kann ein Gel enzymatisch durch Thrombin erzeugt werden. Die Zellen können homogen im Gel enthalten sein oder in Zellaggregaten verbunden von diesem umgeben sein. Der Vorteil eines Hydrogels ist, dass sich damit mechanische und hydrodynamische Bedingungen in Gewebe realistisch simulieren lassen. Die Nährstoffe aus dem Zellkulturmedium können auch leicht durch ein Hydrogel zu den Zellen diffundieren.

Der Abstand zwischen den Säulen und damit die Breite der Öffnungen kann von der Beschaffenheit des Gels, insbesondere dessen Viskosität abhängig gemacht werden. Der Abstand zwischen zwei benachbarten Säulen kann zwischen 50 µm und 500 µm, insbesondere zwischen 100 µm und 300 µm betragen. Dieser Abstand ist typischerweise ausreichend, damit ein Gel und somit die darin enthaltenen Zellen oder Zellaggregate nicht zwischen den Säulen hindurchtreten kann, sondern aufgrund seiner Oberflächenspannung eine stabile Grenzfläche bildet. Weiterhin gilt es zu beachten, dass die Öffnungen nicht zu schmal sind, weil sonst der Austausch zwischen Kanal und Reservoir erschwert sein kann, sodass eine ausreichende Nährstoffversorgung von Zellen nicht sichergestellt werden kann.

Die Einlassöffnung und/oder die Auslassöffnung kann beziehungsweise können in der Oberfläche des Zellkulturträgers ausgebildet sein, insbesondere in der Seite des Deckelements, die jener Seite, an der das Bodenelement angebracht ist, und in der die Vertiefung ausgebildet ist, gegenüberliegt. Der Kanal kann ausgehend von der Einlassöffnung und/oder der Auslassöffnung zumindest teilweise senkrecht zur Oberfläche des Zellkulturträgers verlaufen.

Diese Ausrichtung des Kanals stellt einen weiteren Beitrag zu einer einfachen und effizienten Herstellung eines Zellkulturträgers dar, insbesondere wenn das Deckelement mittels Spritzguss hergestellt wird. Insbesondere ist es bei Spritzguss vergleichsweise einfach möglich, horizontal und/oder vertikal zu einer Oberfläche verlaufende Elemente auszubilden, wohingegen diagonal oder anderweitig verlaufende Elemente schwierig oder unmöglich auszubilden sind. Daher ist die Ausbildung von zumindest teilweise vertikal verlaufenden Kanälen, insbesondere auch in Kombination mit Abschnitten in Form von Gräben, für Spritzgussverfahren vorteilhaft.

Der im Zellkulturträger ausgebildete Kanal kann aus Abschnitten bestehen, die parallel oder senkrecht zur Oberfläche sind. Dies kann einerseits für den Fall zutreffen, in dem der Kanal vollständig als Röhre im Probenträger ausgebildet ist. Insbesondere kann der Kanal auch teilweise aus einer Röhre und teilweise aus einem abgedeckten Graben im Deckelement bestehen. In diesem Fall kann der abgedeckte Graben die parallelen Abschnitte bilden und vertikale Abschnitte können senkrecht zur Oberfläche des Deckelements, als Durchgangslöcher durch das Deckelement verlaufen.

Die Einlassöffnung und/oder die Auslassöffnung kann/können weiterhin über einen Anschluss verfügen. Diese Anschlüsse an den Enden des Kanals können konisch ausgebildet sein. Insbesondere können die Anschlüsse dem Luer-Standard entsprechen, wobei die Anschlüsse einen weiblichen Luer- oder Luerlock- Adapter aufweisen können. Im bestimmungsgemäßen Gebrauch kann dann eine Vorrichtung zum Befüllen des Kanals durch die Anschlüsse einen männlichen Luer- oder Luerlock-Adapter aufweisen.

Durch die Verwendung konischer Anschlüsse, insbesondere von Anschlüssen, die dem Luer-Standard entsprechen, können die Anschlüsse beim Befüllen flüssigkeitsdicht verbunden werden und das Befüllen dadurch einfach und verlässlich durchgeführt werden. Zudem ist der Zellkulturträger mit den meisten Vorrichtungen zum Befüllen kompatibel, da der Luer-Standard in diesem Zusammenhang weit verbreitet ist.

Das Deckelement kann ein Kunststoffträger sein. Insbesondere kann es Kunststoffe wie COC (Cyclo-Olefin Copolymer), COP (Cyclo-Olefin Polymer), PC (Polycarbonat), PS (Polystyrol), PE (Polyethylen), PMMA (Polymethylmetacrylat) oder einen transparenten Thermoplast oder ein Elastomer umfassen. Insbesondere kann das Deckelement durch Spritzguss hergestellt worden sein. Allerdings ist das Deckelement gemäß dieser Beschreibung nicht auf die genannten Materialien und Herstellungsverfahren beschränkt.

Durch die Verwendung der genannten Materialien und Verfahren können die Zellkulturträger kostengünstig, sowie in großer Stückzahl mit konstanter Qualität produziert werden. Dies liegt daran, dass der Spritzguss mit Kunststoffen ein etabliertes und verlässliches Verfahren ist und insbesondere im Fall der genannten Kunststoffe anwendbar ist. Die Verwendung eines transparenten Kunststoffs ist insbesondere vorteilhaft, um optische Untersuchungen im Zellkulturträger vornehmen zu können, beispielsweise via Mikroskopie.

Das Bodenelement kann Kunststoff und/oder Glas umfassen. Als Kunststoffe können COC, COP, PC, PS, PE, PMMA oder andere transparente Kunststoffe, insbesondere Thermoplaste verwendet werden. Insbesondere kann das Bodenelement auch in Form einer Folie ausgebildet sein. Dabei kann das Bodenelement ein Material umfassen, das die Doppelbrechung und die Autofluoreszenz eines Schott-Deckglases (wie D 263 M Schott Glas, Nr. 1,5H (170 +/- 5 µm)) aufweist.

Ein solcher optisch hochwertiger Kunststoff kann mikroskopische Untersuchungen mit hoher Präzision und geringen optischen Imperfektionen ermöglichen, insbesondere bei der Anwendung von Mikroskopie. Beispielsweise kann inverse Mikroskopie am Zellkulturträger durchgeführt werden. Dabei wird ein Objektiv von unten auf den Zellkulturträger gerichtet und die Untersuchung erfolgt durch die Grundfläche, beziehungsweise durch das Bodenelement des Zellkulturträgers hindurch. In diesem Fall sind die genannten hochwertigen optischen Eigenschaften des Bodenelements von Vorteil, um mikroskopische Aufnahmen mit hoher Auflösung und geringen Abbildungsfehlern zu erhalten.

Das Bodenelement kann wie beschrieben durch Verkleben am Deckelement angebracht werden. Insbesondere können in diesem Fall Dispersionsklebstoffe oder Doppelklebebänder verwendet werden. Entsprechend kann der Klebefilm Dispersionsklebstoffe oder Doppelklebebänder umfassen oder daraus bestehen.

Dabei ist das Bodenelement auf eine Art und Weise mit dem Deckelement verbunden, sodass die optischen Eigenschaften des Zellkulturträgers erhalten bleiben und beispielsweise Mikroskopie, insbesondere Fluoreszenzmikroskopie oder inverse Mikroskopie, mit dem Zellkulturträger durchgeführt werden kann. Insbesondere im Bereich des Reservoirs und gegebenenfalls des Kanals muss in diesem Fall eine hohe Auflösung erreicht werden, um die Zellen im Zellkulturträger entsprechend untersuchen oder mikroskopieren zu können. Gleichzeitig sind diese Verfahren im Zusammenhang mit Kunststoffbauteilen etabliert und stellen einen kostengünstigen und effizienten Weg zur verlässlichen Anbringung des Bodenelements an dem Deckelement dar.

An dieser Stelle zeigt sich die Bedeutung, dass der der Klebefilm nicht die Vertiefung im Deckelement bedeckt. Dies hätte zur Folge, dass in der erfindungsgemäßen Verwendung der Klebefilm zwischen dem Reservoir und der Grundfläche des Zellkulturträgers angeordnet wäre. Ein Klebefilm hat allerdings typischerweise keine ausreichenden optischen Eigenschaften, anders als das Bodenelement, um hochauflösende Mikroskopie durch den Klebefilm hindurch durchzuführen, weil Abbildungsfehler induziert würden.

Das Deckelement kann eine Dicke von 0,5 mm bis 5 cm, insbesondere von 5 mm bis 2 cm aufweisen. Dabei ist die Dicke der vertikale Abstand zwischen einer Oberseite und einer Unterseite des Deckelements. Das Bodenelement kann eine Dicke zwischen 1 µm und 2 mm, insbesondere zwischen 1 µm und 300 µm aufweisen. Dabei ist für die Dicke des Bodenelements eine analoge Definition wie für das Deckelement anzuwenden.

Insbesondere kann das Bodenelement als Folie ausgebildet sein, die am Deckelement befestigt ist. Eine geringe Dicke des Bodenelements hat dabei den Vorteil, dass bei inverser Mikroskopie das Objektiv besonders nah an einen zu beobachtenden Bereich im Zellkulturträger herangebracht werden kann. Dies ermöglicht eine verbesserte optische Auflösung.

Zudem beinhaltet die vorliegende Erfindung einen Zellkulturträgerbausatz. Dieser Zellkulturträgerbausatz umfasst:
Ein Deckelement umfassend einen Graben und eine Vertiefung, wobei die Vertiefung mindestens zwei nebeneinander angeordnete Öffnungen aufweist, und wobei der Graben durch die mindestens zwei Öffnungen mit der Vertiefung verbunden ist
einen Klebefilm; und
ein Bodenelement,
wobei das Bodenelement mittels des Klebefilms derart am Probenträger befestigbar ist, sodass die Vertiefung und der Graben durch das Bodenelement abgedeckt sind, und wobei ein Zusammenbau des Zellkulturträgerbausatzes einen beschriebenen Zellkulturträger ergibt.

Hierbei umfasst der Zellkulturträgerbausatz alle Bauteile für einen erfindungsgemäßen Zellkulturträger, sodass ein Anwender den Zellkulturträger entsprechend vielseitig präparieren und anschließend selbst zusammenbauen kann. Beispielweise kann die Vertiefung im Deckelement zunächst mit einem Gel mit Zellen oder Zellaggregaten befüllt werden, danach der Klebefilm am Deckelement angebracht und zuletzt der Zellkulturträger mit dem Bodenelement verschlossen werden. Die im Zusammenhang mit dem Zellkulturträger diskutierten Vorteile treffen daher auch auf den Zellkulturträgerbausatz zu.

Der Zellkulturträger kann aus den besagten Komponenten, also dem Deckelement, dem Bodenelement und dem Klebefilm bestehen.

Der Klebefilm kann auf mindestens einer Seite mit einer Schutzfolie bedeckt sein.

Da bei dem Zellkulturträgerbausatz der Klebefilm separat vorliegen kann, verhindert die Schutzfolie, dass der Klebefilm an anderen Komponenten anhaften kann. Die Schutzfolie kann insbesondere selbst nichtklebend, also an keinem anderen Element als dem Klebefilm haftend, sein und leicht vom Anwender vom Klebefilm zu entfernen sein.

Der Klebefilm kann auf dem Deckelement aufgebracht sein, sodass die Vertiefung und der Graben nicht mit dem Klebefilm bedeckt sind, und wobei die dem Deckelement abgewandte Seite des Klebefilms mit einer Schutzfolie bedeckt ist.

Diese Konfiguration ist besonders anwenderfreundlich, weil es durch die genannte Dimensionierung des Klebefilms in manueller Art und Weise schwierig ist, den Klebefilm akkurat aufzutragen. Ein Anwender kann nun den Probenträger direkt verwenden, ohne den Klebefilm selbst auftragen zu müssen. Für den Zusammenbau können die Schutzfolie entfernt werden und abschließend das Deckelement und das Bodenelement zusammengefügt werden.

Die vorliegende Erfindung beinhaltet darüber hinaus ein Verfahren zum Einbringen von Zellen oder Zellaggregaten in einem Zellkulturträger. Dabei umfasst das Verfahren folgende Schritte:
Bereitstellen eines zuvor beschriebenen Zellkulturträgerbausatzes;
Befüllen der Vertiefung im Deckelement mit einem Gel, das Zellen oder Zellaggregate enthält;
Abdecken der Vertiefung und des Grabens mit dem Bodenelement,
wobei das Deckelement und das Bodenelement flächig miteinander verbunden sind,
wobei die Vertiefung und der Graben durch das Bodenelement abgedeckt sind, und
wobei das Abdecken der Vertiefung nach dem Befüllen der Vertiefung durchgeführt wird.

Nach einer vollständigen Durchführung des beschriebenen Verfahrens erhält man einen zuvor beschriebenen Zellkulturträger, in dessen Reservoir ein Gel mit Zellen oder Zellaggregaten eingebracht ist. Das Verfahren in Verbindung mit dem beschriebenen Zellkulturträgerbausatz bietet dabei die in dieser Beschreibung genannten Vorteile.

Die Öffnungen des Reservoirs können so eng ausgebildet sein, dass die Zellen oder Zellaggregate im Reservoir nicht durch die Öffnungen aus dem Reservoir gelangen können.

Der Vorteil darin, dass es möglich ist, die Zellen in einem definierten Volumen, in diesem Fall dem Reservoir festzuhalten, ohne die Zellen an einer Innenwand des Reservoirs adhärieren zu müssen. Dies ist insofern notwendig, weil gewissen Zelltypen, wie beispielsweise Immunzellen, durch die Adhäsion an eine Oberfläche ihre Eigenschaften verändern können. Auch durch einen Austausch eines Zellmediums können die Zellen nicht aus dem Reservoir geschwemmt werden.

Dabei kann die Größe der Öffnungen an einen verwendeten Zelltyp entsprechend angepasst werden. Beispielsweise kann die Größe der Öffnungen geringer als die Größe der Zellstrukturen sein, sodass die Zellen nicht durch die Öffnungen hindurchtreten können. Bei der Verwendung eines Gels kommt der Effekt hinzu, dass das Gel durch seine Oberflächenspannung eine stabile Grenzfläche ausbilden kann. In diesem Fall kann die Größe der Öffnungen entsprechend an die Eigenschaften des Gels angepasst sein, sodass dessen Oberflächenspannung verhindert, dass es durch die Öffnungen hindurchtreten kann.

In dem beschriebenen Verfahren kann das Gel mittels 3D-Druck in die Vertiefung eingefüllt werden.

Die Verwendung von 3D-Druck zum Befüllen der Vertiefung mit einem Gel hat einerseits den Vorteil, dass die Befüllung automatisiert ablaufen kann. Damit gehen typischerweise eine gute Reproduzierbarkeit und Effizienz, beispielsweise in Form von Zeitersparnis gegenüber anderen, insbesondere manuellen, Methoden einher. Andererseits kann 3D-Druck auch beliebige Strukturen mit hoher Präzision ausbilden und das Gel daher besonders kontrolliert in die Vertiefung eingebracht werden. Der Zellkulturträger insbesondere mit der Verwendung von 3D-Druck kompatibel.

Das beschriebene Verfahren kann weiterhin den Schritt umfassen, dass die Einlassöffnung und die Auslassöffnung des Kanals an eine Perfusion angeschlossen werden, sodass der Kanal von einem Zellmedium durchströmt wird.

Diese Konfiguration stellt eine erfindungsgemäße Verwendung des Zellkulturträgers dar. Das Zellmedium versorgt die Zellen im Reservoir mit Nährstoffen, ohne sie gleichzeitig einer direkten Strömung auszusetzen. Dies erlaubt es schließlich, nicht adhärente Zellen im Reservoir über einen ausreichend langen Zeitraum zu untersuchen. Dazu ist die Versorgung mit Nährstoffen unerlässlich, da die Zellen sonst vorzeitig absterben könnten.

Falls der Zellkulturträger mehr als einen Kanal umfasst, können ein Kanal oder mehrere Kanäle an die Perfusion angeschlossen werden. Es ist ebenfalls möglich, dass die Kanäle an unterschiedliche Perfusionen angeschlossen werden und/oder dass unterschiedliche Kanäle von unterschiedlichen Zellmedien und/oder zu unterschiedlichen Zeitpunkten durchströmt werden.

Weitere Merkmale und Vorteile werden nachfolgend anhand der beispielhaften Figuren erläutert. Dabei zeigen:
- Figur 1: eine schematische Schrägansicht eines Zellkulturträgers gemäß einer ersten Ausführungsform;
- Figuren 2A bis 2D: unterschiedliche Ausführungsformen von im Zellkulturträger ausgebildeten Kanälen und dem Reservoir als Draufsicht;
- Figur 3: eine schematische Schrägansicht eines Zellkulturträgers gemäß einer zweiten Ausführungsform;
- Figur 4A: eine schematische Schrägansicht eines Zellkulturträgers gemäß einer dritten Ausführungsform;
- Figuren 4B und 4C: einen Querschnitt des Zellkulturträgers in Figur 4A entlang zweier unterschiedlicher Schnittachsen;
- Figur 5: einen Zellkulturträgerbausatz; und
- Figuren 6A bis 6C: ein Verfahren zum Einbringen von Zellen oder Zellaggregaten in einen Zellkulturträger.

Im Folgenden und in den Figuren werden in den verschiedenen Ausführungsbeispielen, sofern nicht anders spezifiziert, die gleichen Bezugszeichen für gleiche oder entsprechende Elemente verwendet.

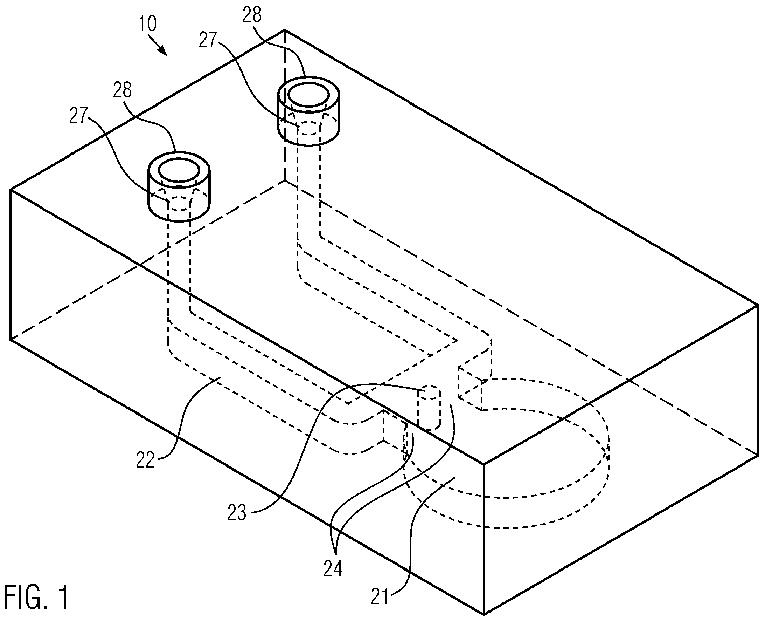

Figur 1 illustriert die verschiedenen Elemente eines Zellkulturträgers 10 gemäß der vorliegenden Erfindung. Dabei ist der Zellkulturträger 10 in einer Schrägansicht dargestellt. In diesem Fall weist der Zellkulturträger 10 die Form eines Quaders mit einer planen Unterseite auf und umfasst ein oder besteht aus einem transparenten Material, insbesondere einem der in dieser Beschreibung genannten Kunststoff COC, COP, PC, PS, PE, PMMA oder einem transparenten Thermoplast oder einem Elastomer. Der gezeigte Zellkulturträger 10 weist eine rechteckige Grundfläche auf, die dessen Unterseite entspricht. Die Grundfläche des Zellkulturträgers 10 kann grundsätzlich auch andere Formen haben, beispielsweise anderweitig vieleckig oder mit abgerundeten Ecken und/oder Kanten. Der Unterseite gegenüber liegt die Oberseite des Zellkulturträgers 10.

Eine plane Seite ist dabei insbesondere bei einer optischen Untersuchung von Vorteil. Beispielsweise in der Mikroskopie wird eine plane Unterseite benötigt, um optische Abbildungsfehler, wie Astigmatismus oder sphärische Aberrationen, zu reduzieren oder zu vermeiden.

Im Inneren des Zellkulturträgers 10 ist das Reservoir 21 in Form eines zylindrischen, d.h. im Querschnitt kreisförmigen und mit einem Boden und einer Decke versehenen, Hohlraums ausgebildet. Wie nachfolgend beschrieben, ist die Form des Hohlraums nicht auf eine zylindrische Geometrie beschränkt. Das zylindrische Reservoir 21 besitzt eine Mantelfläche, die im Folgenden als Seitenwand bezeichnet wird. In dieser Seitenwand sind zwei benachbarte Öffnungen 24 angeordnet, die durch eine dazwischen angeordnete Barriere oder Säule 23 getrennt sind. Diese Säule 23 weist im vorliegenden Beispiel einen runden Querschnitt auf, allerdings sind auch andere Querschnittsformen denkbar, beispielsweise elliptisch, rechteckig oder anderweitig vieleckig.

Weiterhin umfasst der Zellkulturträger 10 einen Kanal 22, der im Inneren des Zellkulturträgers 10 als Röhre ausgebildet ist. In der Oberseite des Zellkulturträgers 10 sind eine Einlassöffnung 27 und eine Auslassöffnung 27 jeweils in Form eines Lochs ausgebildet. Da die Einlassöffnung 27 und die Auslassöffnung 27 grundsätzlich sowohl als Einlass, als auch als Auslass dienen können, werden beide mit demselben Bezugszeichen versehen. Zudem sind die beiden Löcher mit einem Anschluss 28 versehen. Damit verläuft der Kanal 22 innerhalb des Zellkulturträgers 10 zwischen der Einlassöffnung 27 und der Auslassöffnung 27. Ausgehend von der Einlassöffnung 27 und der Auslassöffnung 27 verläuft der Kanal zunächst senkrecht zur Oberseite durch den Zellkulturträger 10. In einer Ebene parallel zur Grundfläche, in der auch das Reservoir 21 angeordnet ist, verläuft der Kanal 22 horizontal durch den Zellkulturträger 10.

Genauer gesagt entspricht in diesem Beispiel der Durchmesser des Kanals 22 der Höhe des Reservoirs 21. Die Höhe des Reservoirs 21 ist dabei der Abstand zwischen dem Boden und der Decke und der Kanal 22 und das Reservoir 21 liegen auf derselben Ebene innerhalb des Zellkulturträgers 10. Allerdings müssen der Durchmesser des Kanals 22 und die Höhe des Reservoirs 21 nicht identisch sein. Die Höhe des Reservoirs 21 kann größer sein als der Durchmesser des Kanals 22 oder umgekehrt.

Der Kanal 22 ist durch die beiden benachbarten Öffnungen 24 mit dem Reservoir 21 verbunden. Eine durch die Kanal 22 strömende Flüssigkeit kann daher durch die Öffnungen 24 in das Reservoir 21 eindringen. Dabei sind die Öffnungen 24 derart angeordnet, dass sie senkrecht zur Strömungsrichtung einer im Kanal 22 strömenden Flüssigkeit ausgerichtet sind. Auf diese Weise kann erreicht werden, dass keine direkte Strömung vom Kanal 22 in das Reservoir 21 gerichtet ist, sondern ein Austausch zwischen Reservoir 21 und Kanal 22 lediglich durch Diffusion stattfindet. Zu diesem Effekt kann weiterhin beitragen, dass die Breite der Öffnungen 24 typischerweise kleiner sein kann als der Durchmesser des Kanals 22. Im Allgemeinen sind die Öffnungen 24 seitlich im Kanal 22 angebracht, sodass sie nicht in Strömungsrichtung liegen. Eine zur Strömungsrichtung senkrechte Ausrichtung stellt einen Spezialfall dar.

Die in der Figur gezeigten Anschlüsse 28 sind konisch ausgebildet und entsprechen insbesondere dem Luer-Standard. Dabei sind beide der Anschlüsse 28 weibliche Luer-Anschlüsse. Beim Anschließen einer entsprechenden Vorrichtung (nicht gezeigt), um den Kanal 22 mit einem Medium zu durchspülen, vereinfacht dies eine flüssigkeitsdichte und effiziente Verbindung zwischen den Anschlüssen 28 und der Vorrichtung. Entsprechend kann die Vorrichtung mit einem männlichen Luer- oder Luerlock-Adapter ausgestattet sein.

Die konkrete Auswahl von Höhe, Länge und Breite des Zellkulturträgers 10, sowie von Form, Volumen und konkreter Ausführungsform des Reservoirs 21 und des Kanals 22 können gemäß der jeweiligen Verwendung des Zellkulturträgers 10 festgelegt werden. So kann beispielsweise eine rechteckige Form, die einem typischen in der Mikroskopie verwendeten Objektträger nachempfunden ist, bei der mikroskopischen Untersuchung des Zellkulturträgers 10 von Vorteil sein. Form und Volumen des Reservoirs 21, sowie die Anzahl, Breite und Abstand der Öffnungen 24 können beispielsweise auf die darin einzubringenden Zellen oder Zellaggregaten optimiert sein. Ebenso ist der Zellkulturträger 10 nicht auf den einen gezeigten Kanal 22 beschränkt. Es können auch ein zweiter Kanal oder mehr als zwei Kanäle vorhanden sein, wie in nachfolgend beschriebenen Ausführungsbeispielen zu sehen ist.

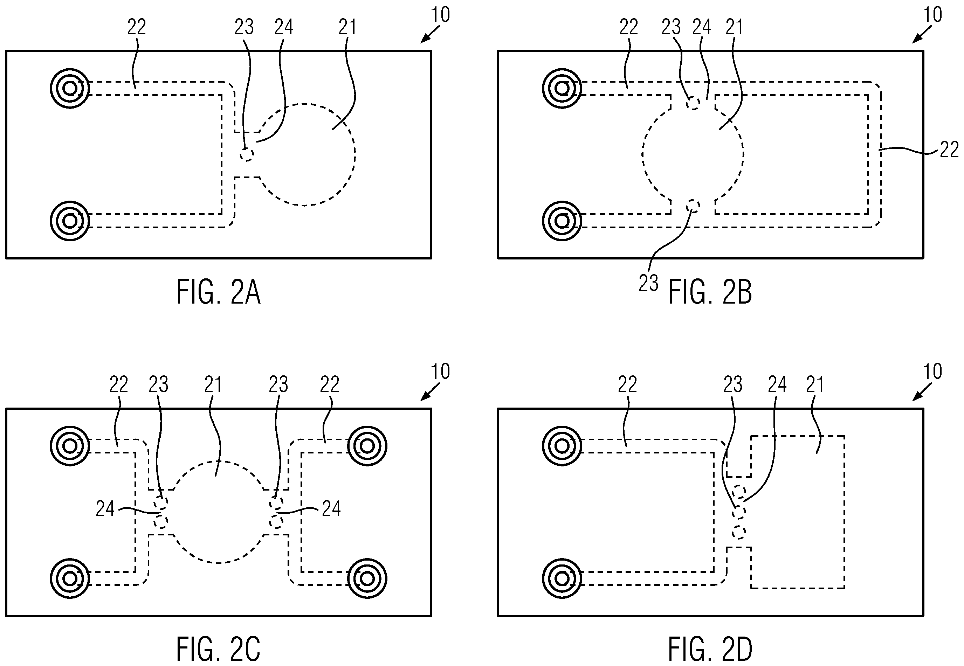

Die Figuren 2A bis 2D zeigen verschiedene Ausführungsformen von im Zellkulturträger 10 ausgebildeten Kanälen 22 und einem Reservoir 21 als Draufsicht. Der Zellkulturträger 10 selbst weist in diesem Fall eine rechteckige Grundfläche auf, allerdings ist dies nicht als einschränkend zu verstehen. Die gezeigten Ausführungsbeispiele für das Reservoir 21 und die Kanäle 22 sind mit anderen Formen des Zellkulturträgers 10 kombinierbar. Diese Ausführungsformen sind insbesondere mit den in den Figuren 1, 3 und 4A gezeigten Ausführungsbeispielen eines Zellkulturträgers 10 kombinierbar.

Gemäß dem in Figur 2A gezeigten Ausführungsbeispiel umfasst der Zellkulturträger 10 ein Reservoir 21 mit einem kreisförmigen Querschnitt, sowie einen Kanal 22. Dieser Kanal 22 ist mit dem Reservoir 21 durch zwei benachbarte Öffnungen 24 verbunden. Die beiden Öffnungen 24 sind dabei durch eine einzelne Säule 23 getrennt. Im gezeigten Beispiel weist der Kanal 22 eine U-Form auf, bei der die Einlassöffnung und die Auslassöffnung des Kanals 22 auf der gleichen Seite des Reservoirs 21 angeordnet sind. Es auch andere Anordnungen des Kanals 22 bezüglich des Reservoirs 21 möglich. Beispielsweise kann ein Kanal 22 geradlinig verlaufen und mit dem Reservoir 21 verbunden sein, wobei die Einlassöffnung und die Auslassöffnung des Kanals 22 auf unterschiedlichen Seiten des Reservoirs 21 angeordnet sind.

In Figur 2B weist das Reservoir 21 einen kreisförmigen Querschnitt auf. Im Zellkulturträger 10 ist ein Kanal 22 ausgebildet, der das Reservoir 21 an zwei gegenüberliegenden Stellen, den sogenannten Kontaktpunkten, kontaktiert. An diesen Kontaktpunkten ist der Kanal 22 mit dem Reservoir 21 durch die Öffnungen 24 verbunden. Eine derartige Bezeichnung kann auch auf die anderen Ausführungsformen angewandt werden. Es ist allerdings auch möglich, dass die Kontaktpunkte zwischen dem Kanal 22 und dem Reservoir 21 an anderen als den gezeigten Stellen liegen. Die Kontaktpunkte können in einer anderen Konfiguration am Reservoir 21 angeordnet sein, solange sie die in dieser Beschreibung genannte Definition gegenüberliegender Stellen berücksichtigen. In der Seitenwand des Reservoirs 21 befinden sich an den besagten Kontaktpunkten jeweils zwei benachbarte Öffnungen 24, die durch eine Säule 23 getrennt sind. Dieses Beispiel zeigt eine einzelne Säule 23, es kann aber auch eine Mehrzahl von Säulen 23, sowie eine Mehrzahl von Öffnungen 24 vorhanden sein.

Figur 2C zeigt ein weiteres Ausführungsbeispiel des besagten Probenträgers 10 umfassend ein Reservoir 21 mit einem kreisförmigen Querschnitt und zwei Kanäle 22. Jeder der beiden Kanäle 22 ist mit dem Reservoir 21 durch drei benachbarte Öffnungen 24 verbunden. Diese drei Öffnungen 24 sind durch zwei Säulen 23 voneinander getrennt. Die Öffnungen 24 in der Seitenwand des Reservoirs 21 sind dabei an gegenüberliegenden Stellen ausgebildet. Weiterhin weist jeder der beiden Kanäle 22 eine U-Form auf.

Anstelle eines kreisförmigen Querschnitts kann das Reservoir 21 in den gezeigten Ausführungsbeispielen beispielsweise einen elliptischen oder rechteckigen Querschnitt aufweisen. Insbesondere ist die Kombination von zwei Kanälen 22 auch nicht auf die gezeigte Anordnung von 21 und Kanälen 22 beschränkt. Beispielsweise müssen die Öffnungen nicht an gegenüberliegenden Stellen in der Seitenwand des Reservoirs 21 ausgebildet sein. Ebenfalls können die Kanäle 22 auch in unterschiedlichen Formen ausgebildet sein, beispielsweise kann der erste Kanal 22 U-förmig verlaufen, während der zweite Kanal 22 geradlinig ausgebildet ist.

Ein Vorteil dieser Ausführungsform mit zwei Kanälen ist ein beschleunigter Austausch eines Mediums oder einer Flüssigkeit zwischen den Kanälen 22 und dem Reservoir 21, insbesondere gegenüber einer Ausführungsform mit einem Kanal 22. Zusätzlich wird die Möglichkeit einer Ausbildung von Totbereichen weiter reduziert, in denen kein Austausch stattfindet oder nur auf langen Zeitskalen gegenüber anderen Bereichen des Reservoirs 21.

Das Reservoir 21 in Figur 2D weist einen rechteckigen Querschnitt auf und insgesamt vier benachbarte Öffnungen 24 in seiner Seitenwand. Diese vier Öffnungen 24 sind dabei durch drei Säulen 23 voneinander getrennt. Ein einzelner Kanal 22, der eine U-Form aufweist, kontaktiert das Reservoir 21 an den insgesamt vier benachbarten Öffnungen 24.

Insbesondere bei einem rechteckigen Reservoir 21 ist es möglich, dass der Kanal 22 über einen längeren Bereich parallel zum Reservoir 21 verläuft. Entsprechend kann eine größere Zahl von Öffnungen 24 in der Seitenwand des Reservoirs 21 realisiert werden. Dies trägt wie beschrieben zu einem schnelleren und verbesserten Austausch eines Mediums zwischen dem Kanal 22 und dem Reservoir 21 bei.

Die in den Figuren 2A bis 2D gezeigten Beispiele sind nicht auf diese konkreten Kombinationen beschränkt. So ist die Form des Querschnitts des Reservoirs 21 frei mit der Anzahl und Anordnung eines oder mehrerer Kanäle 22, sowie der Anzahl der Öffnungen 24 kombinierbar. Beispielsweise kann ein rechteckiges Reservoir 21 zusammen mit zwei Kanälen 22 im Zellkulturträger 10 ausgebildet sein. Auch die Zahl der Öffnungen ist nicht an die Zahl der Kanäle 22 oder die Querschnittsform des Reservoirs 21 gebunden.

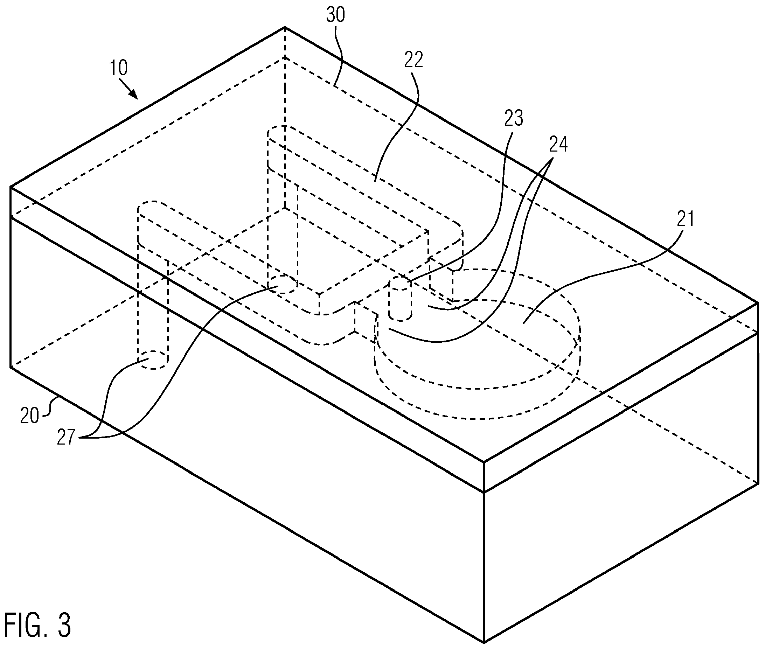

Figur 3 stellt die Schrägansicht eines Zellkulturträgers 10 dar. Der Zellkulturträger 10 umfasst hierbei ein Deckelement 20 und ein Bodenelement 30. Die weiteren gezeigten Elemente des Zellkulturträgers 10 entsprechen jenen aus dem Ausführungsbeispiel gemäß Figur 1.

In diesem Ausführungsbeispiel unterscheidet sich die Ausbildung des Kanals 22 und des Reservoirs 21 im Zellkulturträger 10 vom Ausführungsbeispiel gemäß Figur 1 dahingehend, dass ein Graben oder eine rillenartige Vertiefung an einer Oberfläche des Deckelements 20 ausgebildet ist, die dem Bodenelement zugewandt ist. Das Bodenelement 30 deckt dabei den Graben ab und bildet einen vollständigen Kanal 22, der innerhalb des Zellkulturträgers 10 verläuft. Wie im Beispiel gezeigt, verläuft der Kanal 22 jeweils am Ende des Grabens vertikal durch den Zellkulturträger 10 hindurch, sodass die Einlassöffnung 27 und die Auslassöffnung 27 auf einer Seite des Deckelements 20 ausgebildet sind, die der Seite, in der der Graben ausgebildet ist, gegenüberliegt. Anders gesagt befinden sich die Einlassöffnung 27 und die Auslassöffnung 27 auf der dem Bodenelement 30 abgewandten Seite des Deckelements 20.

Weiterhin umfasst das Deckelement 20 eine Vertiefung, die in der gleichen Seite der Oberfläche wie auch der Graben ausgebildet ist. Die Vertiefung ist also in der dem Bodenelement 30 zugewandten Seite des Deckelements 20 ausgebildet. Wie das Reservoir 21 im vorangegangenen Ausführungsbeispiel weist auch die Vertiefung hier zwei benachbarte Öffnungen 24 auf, die durch eine Säule 23 dazwischen getrennt sind. Der Graben ist durch die beiden Öffnungen 24 mit der Vertiefung verbunden. Die Vertiefung und der Graben können eine gleiche Tiefe im Deckelement 20 aufweisen, allerdings kann auch der Graben eine größere Tiefe als die Vertiefung aufweisen oder umgekehrt.

Der Graben ist mit dem Bodenelement 30 abgedeckt. Insbesondere kann es sich bei dem Bodenelement 30 um eine transparente Folie, insbesondere aus den Materialien COC, COP, PC, PS, PE, PMMA oder einem anderen transparenten Kunststoff oder einem Thermoplast, handeln. Diese Folie kann dabei insbesondere die Autofluoreszenz und die Doppelbrechung eines Schott-Deckglases aufweisen. Dabei wird der Graben derart abgedeckt, dass eine durch den Kanal 22 strömende Flüssigkeit nicht an den Gräben austreten kann. Die Abdeckung mit dem Bodenelement 30 ist also flüssigkeitsdicht.

Darüber hinaus wird durch das Bodenelement 30 auch gleichzeitig die Vertiefung abgedeckt, sodass auch diese flüssigkeitsdicht verschlossen ist. Durch die Abdeckung der Vertiefung mit dem Bodenelement wird das Reservoir 21 innerhalb des Zellkulturträgers 10 gebildet.

Zudem wird durch das Abdecken mit dem Bodenelement 30, also durch das Verbinden von Deckelement 20 und Bodenelement 30 der vollständige Zellkulturträger 10 gebildet.

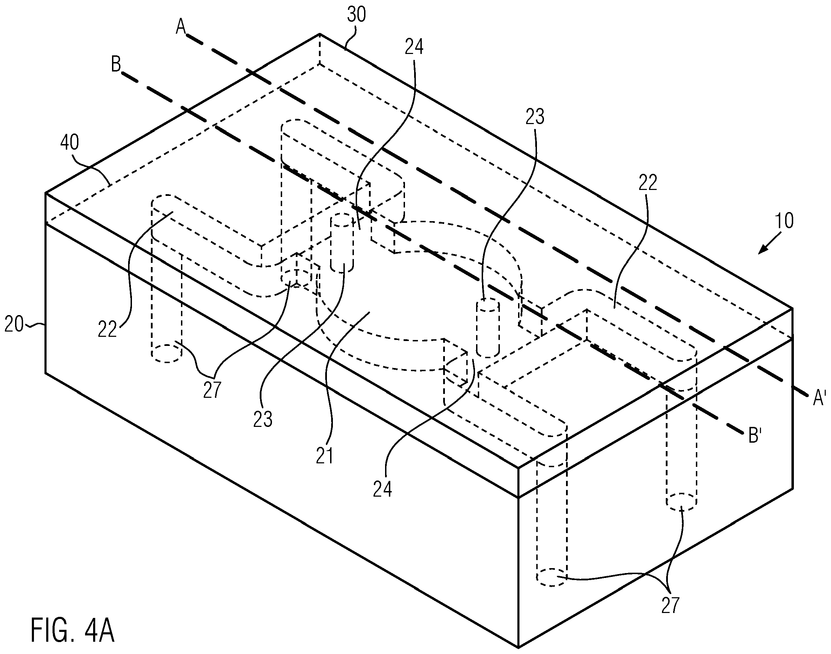

Figur 4A zeigt eine Schrägansicht eines Zellkulturträgers 10 gemäß einem weiteren Ausführungsbeispiel.

Der gezeigte Zellkulturträger 10 umfasst drei Elemente, ein Deckelement 20, ein Bodenelement 30 und einen zwischen dem Deckelement 20 und dem Bodenelement 30 angeordneten Klebefilm 40. Das Deckelement 20 umfasst eine Vertiefung, und zwei Gräben. Beide Gräben sind mit der Vertiefung durch zwei benachbarte Öffnungen 24 verbunden. Die beiden Paare benachbarter Öffnungen 24 sind dabei an gegenüberliegenden Stellen in der Vertiefung angeordnet. Ausgehend von den Gräben verlaufen Kanäle 22 weiterhin senkrecht zur Oberfläche und in Form einer Röhre durch das Deckelement 20 hindurch. Auf der der Vertiefung abgewandten Seite des Deckelements 20 sind für jeden Kanal 22 jeweils eine Einlassöffnung 27 und eine Auslassöffnung 27 in Form eines Lochs in der Oberfläche des Zellkulturträgers 10 ausgebildet.

Die beiden Paare von Öffnungen 24 sind jeweils durch eine dazwischen angeordnete zylindrische Säule 23 getrennt. Dabei besitzt die Säule 23 eine Säulenhöhe, wobei die Säulenhöhe die Länge des Zylinders definiert. Gleichsam besitzt auch die Vertiefung eine Höhe, die als der vertikale Abstand zwischen dem Boden der Vertiefung und der Oberfläche des Deckelements 20 definiert ist. Im gezeigten Beispiel ist die Säulenhöhe beider Säulen 23 größer als die Höhe der Vertiefung, sodass die Säulen 23 über die Oberfläche des Deckelement 20 hinausragen.

Wie beschrieben ist zwischen dem Bodenelement 30 und dem Deckelement 20 ein Klebefilm 40 angeordnet, der das Deckelement 20 mit dem Bodenelement 30 flächig verbindet und verklebt. Dabei ist der Klebefilm 40 derart ausgebildet, dass er eine Seite der Oberfläche des Deckelements 20 bedeckt, allerdings die Gräben, die Vertiefung, die Öffnungen 24 und die Säulen 23 nicht bedeckt. Der Klebefilm 40 kann dabei die gesamte beschriebene Fläche bedecken, allerdings ist es auch denkbar, dass der Klebefilm 40 nur Teile dieser Fläche bedeckt. Entscheidend ist hierbei, dass das Deckelement 20 und das Bodenelement 30 stabil durch den Klebefilm 40 miteinander verklebt werden. Weiterhin ist das Deckelement 30 auch in diesem gezeigten Beispiel dazu ausgelegt, die Gräben und die Vertiefung abzudecken und flüssigkeitsdicht zu verschließen.

Die Differenz der Säulenhöhe und der Höhe der Vertiefung kann insbesondere einer Dicke des Klebefilms 40 entsprechen. Die Dicke des Klebefilms 40 ist hierbei seine vertikale Ausdehnung senkrecht zur Oberfläche des Deckelements 20. Dies hat den Vorteil, dass eine dichte Verbindung zwischen dem Deckelement 20 und dem Bodenelement 30 hergestellt werden kann, weil die Säulen 23 mit dem Bodenelement 30 in Berührung kommen und eine dichte Verbindung herstellen. Wäre die Säulenhöhe kleiner als die Summe der Höhe der Vertiefung und der Dicke des Klebefilms 40, könnten aufgrund der Dicke des Klebefilms 40 Spalten oder andere Lücken zwischen dem Deckelement 20 und dem Bodenelement 30 entstehen, durch die Zellen oder Zellaggregaten im Reservoir 21 entweichen können und/oder durch die das Zellmedium, das durch die Kanäle 22 strömen kann, austreten kann. Es ist auch möglich, dass die Säulenhöhe größer ist als die Summe der Höhe der Vertiefung und der Dicke des Klebefilms 40. In diesem Fall kann eine flüssigkeitsdichte Verschließung der Gräben und der Vertiefung durch das Bodenelement 30 durch einen erhöhten Anpressdruck erzielt werden. Dies ist allerdings auch nicht mehr möglich, falls die Säulen 23 zu weit über die Oberfläche des Deckelement 20 hinausragen.

Wie im vorherigen Ausführungsbeispiel werden die Gräben und die Vertiefung durch das Bodenelement 30 abgedeckt. Dadurch werden die vollständigen Kanäle 22 und das Reservoir 21 im Inneren des Zellkulturträgers 10 gebildet.

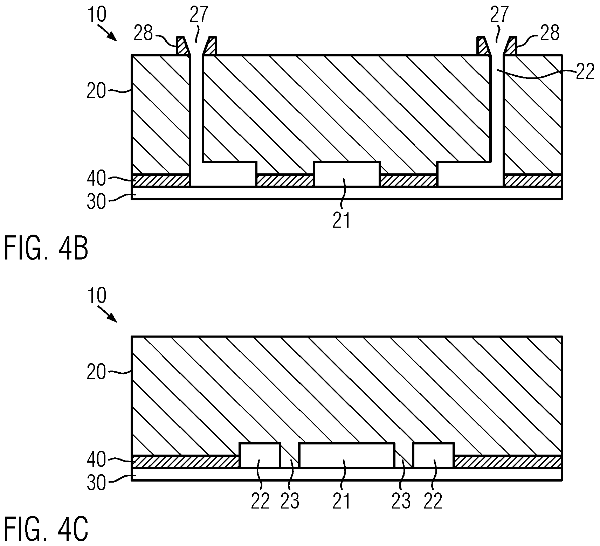

Zuletzt zeigt die Figur zwei Achsen A-A' und B-B', auf die in den nachfolgenden Figuren 4B und 4C Bezug genommen wird.

Figur 4B zeigt einen Querschnitt des Zellkulturträgers 10 aus Figur 4A, wobei der Schnitt entlang der in Figur 4A gezeigten Achse A-A' durchgeführt wurde. Hierbei ist das Deckelement 20 oberhalb des Bodenelement 30 dargestellt und der Klebefilm 40 ist zwischen dem Deckelement 20 und dem Bodenelement 30 angeordnet. Folglich befinden sich die Gräben und die Vertiefung an der unteren Seite des Deckelements 20. An der Oberseite sind die Einlassöffnung 27 oder die Auslassöffnung 27 des ersten Kanals 22, sowie die Einlassöffnung 27 oder die Auslassöffnung 27 des zweiten Kanals 22 angeordnet. Weiterhin verfügen die gezeigten Einlassöffnungen 27 beziehungsweise Auslassöffnungen 27 jeweils über einen konischen Anschluss 28, der insbesondere dem Luer-Standard entsprechen kann.

Je nach Ausführungsform der Vertiefung muss diese nicht zwingend im gezeigten Querschnitt auftreten. Beispielsweise kann der Durchmesser des kreisförmigen Querschnitts der Vertiefung so klein ausgebildet sein, dass im Querschnitt entlang der Achse A-A' kein Segment der Vertiefung mehr enthalten ist.

Wie dargestellt bedeckt der Klebefilm 40 die untere Oberfläche des Deckelements 20, wobei die Gräben und die Vertiefung nicht von dem Klebefilm 40 bedeckt sind. Die Gräben und die Vertiefung werden durch das Bodenelement 30 abgedeckt. Das Bodenelement 30 bildet dabei den unteren Abschluss des Zellkulturträgers 10. Durch die Abdeckung der Vertiefung mit dem Bodenelement 30 wird das Reservoir 21 im Zellkulturträger 10 gebildet.

Dass das Bodenelement 30 den unteren Abschluss des Zellkulturträgers 10 bildet, stellt dessen bestimmungsgemäße Verwendung dar. Beispielsweise kann inverse Mikroskopie am Zellkulturträger 10 durchgeführt werden, wobei ein Objektiv von unten durch das Bodenelement 30 hindurch auf das Reservoir 21 im Zellkulturträger 10 gerichtet wird.

Figur 4C zeigt ebenfalls einen Querschnitt des Zellkulturträgers 10 aus Figur 4A, wobei der Schnitt entlang der in Figur 4A gezeigten Achse B-B' durchgeführt wurde. Im Unterschied zu Figur 4B sind in diesem Fall die Säulen 23 zu sehen. Wie dargestellt sind die Säulen 23 im Deckelement 20 ausgebildet, und ragen über die Oberfläche des Deckelements 20 hinaus. Die Säulen 23 weisen also eine größere Höhe als das Reservoir und die Gräben auf. Darüber hinaus berühren die Säulen 23 das Bodenelement 30, entsprechend kann auf dem Säulen 23 kein Klebefilm 40 angeordnet sein. Durch die Abdeckung der Vertiefung und der Gräben mit dem Bodenelement 30 werden das Reservoir 21 und die Kanäle 22 im Zellkulturträger 10 gebildet.

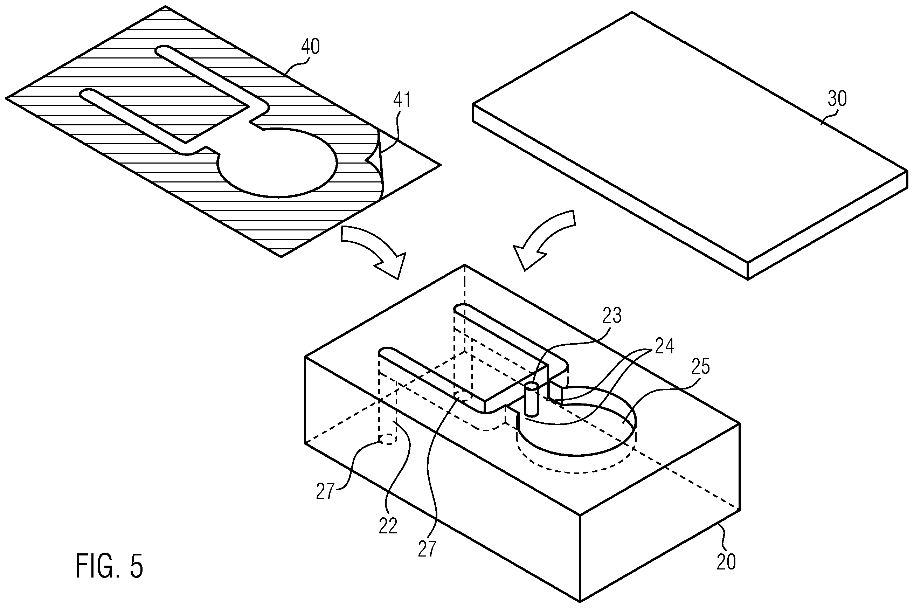

Ein Zellkulturträgerbausatz gemäß der vorliegenden Erfindung ist in Figur 5 dargestellt. Dieser umfasst zunächst ein Deckelement 20 mit einer darin ausgebildeten Vertiefung 25 mit einem kreisförmigen Querschnitt, zwei benachbarten Öffnungen 24 in der einer Seitenwand der Vertiefung 25, wobei die Öffnungen 24 durch eine zylindrische Säule 23 getrennt sind, sowie einen Graben. Die Vertiefung 25 und der Graben sind in der gleichen Seite der Oberfläche des Deckelements 20 ausgebildet. An den beiden Enden des Grabens schließt sich jeweils ein Kanal 22 an, der als Röhre im Deckelement 20 ausgebildet ist und senkrecht als Durchgangsöffnung durch das Deckelement 20 verläuft. In der der Vertiefung 25 abgewandten des Deckelements 20 sind zudem eine Einlassöffnung 27 und eine Auslassöffnung 27 des Kanals 22 angeordnet. Wie im Ausführungsbeispiel gemäß Figur 4A ragt die Säule 23 dabei über die Oberfläche des Bodenelements 20 hinaus.

Separat zum Deckelement 20 umfasst der Bausatz weiterhin einen Klebefilm 40, der auf der Oberfläche des Deckelements 20, an der auch die Vertiefung 25 und der Graben ausgebildet sind, angebracht werden kann. Dabei umfasst der Klebefilm 40 eine Aussparung, deren Form dem Umriss der Vertiefung 25 und des Grabens in der Oberfläche des Deckelements 20 entspricht. Weiterhin kann der Klebefilm 40 auf mindestens einer Seite, insbesondere auf beiden Seiten, von einer Schutzfolie 41 bedeckt sein. Diese Schutzfolie 41 hat den Zweck, dass der Klebefilm 40 separat gelagert werden kann und dabei an keinem anderen Objekt anhaftet. Vor dem Anbringen des Klebefilms 40 auf dem Deckelement 20 müsste die Schutzfolie 41 entfernt werden.

Zuletzt umfasst der Bausatz auch ein Bodenelement 30, das auf dem Deckelement 20 und dem Klebefilm 40 aufgebracht werden kann. Insbesondere kann das Bodenelement 30 eine Folie sein.

Gemeinsam ergeben die beschriebenen Teile einen beschriebenen vollständigen Zellkulturträger 10, der für die in dieser Beschreibung geschilderte Verwendung vorgesehen ist und die genannten Eigenschaften aufweist.

Für die konkreten Eigenschaften des Deckelements 20 und des Bodenelements 30 gelten insbesondere die in dieser Beschreibung dargelegten Überlegungen hinsichtlich der konkreten Formgebung und Wahl der Materialien. Weiterhin gelten insbesondere für das Bodenelement 20 die in dieser Beschreibung genannten Kombinationsmöglichkeiten, beispielsweise hinsichtlich der Zahl und Anordnung der Kanäle 22 und die Form der Vertiefung 25. In diesem Fall muss die Form des Klebefilms 40 entsprechend angepasst werden, damit beim Zusammenbau des Bausatzes die Gräben, die Vertiefung 25, die Öffnungen 24 und die Säulen 23 auch für andere Ausführungsformen des Deckelements 20 nicht mit dem Klebefilm 40 bedeckt werden. Die konkrete Ausgestaltung des Zellkulturträgerbausatzes ist daher nicht auf eine Kombination des gezeigten Deckelements 20 mit einem entsprechenden Klebefilm 40 und einem Deckelement 30 beschränkt.

Für den Zusammenbau des Bausatzes wird zunächst der Klebefilm 40 auf das Deckelement 20 aufgebracht, und zwar auf die Seite, in der die Vertiefung 25 und der Graben ausgebildet sind. Falls der Klebefilm beidseitig mit einer Schutzfolie 41 bedeckt ist, muss die Schutzfolie 41 zunächst auf einer Seite entfernt werden. Wenn der Klebefilm 40 aufgebracht wurde, bedeckt der Klebefilm 40 weder die Vertiefung 25, noch die Öffnungen 24, noch die Säulen 23, noch den Graben. Abschließend wird das Bodenelement 30 auf das Deckelement 20 und den Klebefilm 40 aufgebracht, sodass das Deckelement 20 und das Bodenelement 30 flächig miteinander verbunden und verklebt sind und der Graben und die Vertiefung 25 durch das Bodenelement 30 abgedeckt werden. Auf diese Weise werden der Kanal 22 und das Reservoir 21 gebildet. Nach dem Zusammenbau des Bausatzes erhält man einen vollständigen Zellkulturträger 10 gemäß der vorliegenden Erfindung.

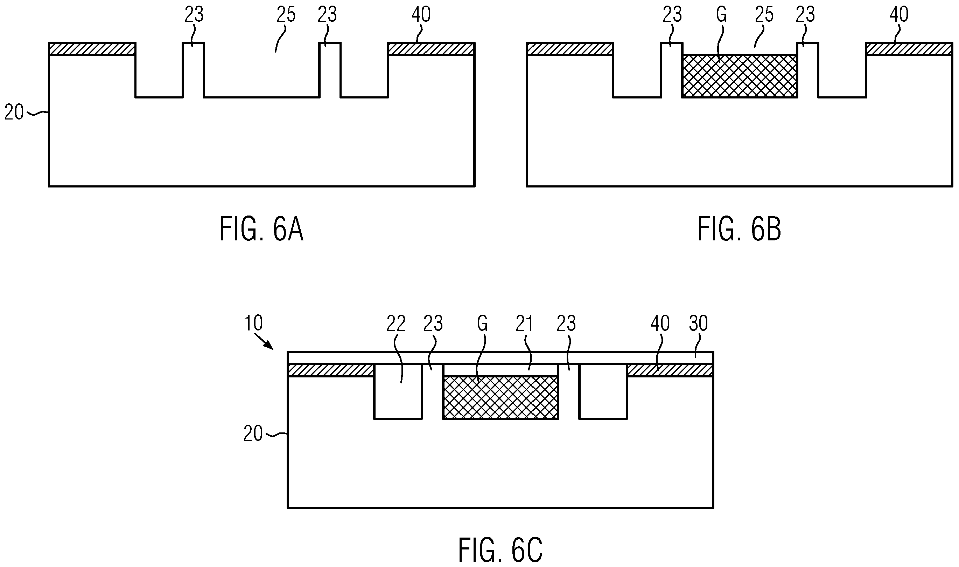

In den Figuren 6A bis 6C ist ein Verfahren zum Einbringen von Zellen oder Zellaggregaten in einen Zellkulturträger 10 dargestellt. Dabei zeigen die einzelnen Figuren einen Querschnitt des in Figur 5 dargestellten Zellkulturträgerbausatzes nach verschiedenen Schritten des Verfahrens. Der Schnitt wurde dabei analog zur Achse B-B' in Figur 4A durchgeführt. Demgemäß umfasst der Zellkulturträgerbausatz zunächst ein Deckelement 20 mit einer Vertiefung 25 und mindestens einem Graben in der Oberfläche des Deckelements 20. Weiterhin umfasst die Vertiefung 25 mindestens zwei Paare von jeweils zwei Öffnungen in ihrer Seitenwand, die an gegenüberliegenden Stellen in der Seitenwand angeordnet und durch eine Säule 23 getrennt sind. Entsprechend umfasst das Deckelement 20 zwei Säulen 23, die im gezeigten Querschnitt zu sehen sind. Die Säulen 23 ragen über die Oberfläche des Deckelements 20 hinaus. Auf dem Deckelement 20 ist ein Klebefilm 40 aufgebracht, wobei der Klebefilm 40 nicht die Vertiefung 25, die Gräben und die Säulen 23 bedeckt.

Im ersten Schritt des Verfahrens wird zunächst, wie in Figur 6A gezeigt, ein Deckelement 20 eines Zellkulturträgerbausatzes bereitgestellt, wobei der Klebefilm 40 bereits auf dem Deckelement 20 angebracht ist.

Gemäß Figur 6B wird daraufhin die Vertiefung 25 mit einem Gel G, das Zellen oder Zellaggregate enthält, befüllt. Das Gel G kann so weit eingefüllt werden, dass die Vertiefung 25 vollständig befüllt ist. Dies entspricht einer maximalen Füllhöhe. Dieses jedoch nicht zwangsläufig der Fall. Ebenso ist es möglich, dass die Vertiefung 25 zu einer beliebigen Füllhöhe unterhalb der maximalen Füllhöhe befüllt wird, wie in Figur 6B gezeigt. Es ist zudem nicht zwingend erforderlich, dass die gesamte Querschnittsfläche der Vertiefung 25 für die Befüllung mit dem Gel G ausgenutzt wird. So kann beispielsweise eine geringe Menge Gel G in das Zentrum der Vertiefung 25 eingebracht werden, wobei ein Durchmesser der auf diese Weise eingebrachten Gelstruktur kleiner ist als der Durchmesser der Vertiefung 25 und/oder die Höhe der Vertiefung 25. Das Befüllen der Vertiefung 25 mit dem Gel G kann insbesondere mittels 3D-Druck, beziehungsweise unter Verwendung eines 3D-Druckers durchgeführt werden. Dazu wird eine Nadel des 3D-Druckers von oben in die Vertiefung 25 eingeführt und das Gel G entsprechend in die Vertiefung 25 hinein gedruckt. Ein Vorteil der Verwendung eines 3D-Druckers ist hierbei die präzise oder detaillierte und reproduzierbare Ausbildung einer Gelstruktur in der Vertiefung 25.

In einem nächsten Schritt wird die Vertiefung 25 mit dem Bodenelement 30 abgedeckt, sodass das Reservoir 21 gebildet wird. Gleichzeitig werden auch die Gräben abgedeckt, sodass die Kanäle 22 gebildet werden und nun vollständig innerhalb des Zellkulturträgers 10 verlaufen. Dieser Schritt ist in Figur 6C dargestellt.

Es sei angemerkt, dass zu Beginn des Verfahrens der Klebefilm 40 noch nicht auf dem Deckelement 20 aufgebracht sein muss. Stattdessen kann der Klebefilm 40 auch in einem eigenen Verfahrensschritt auf dem Deckelement 20 angebracht werden. Dieser Verfahrensschritt muss dabei vor dem finalen Verschließen des Zellkulturträgers 10 durchgeführt werden.

Das beschriebene Verfahren kann zusammen mit dem in dieser Beschreibung aufgeführten Zellkulturträgerbausatz durchgeführt werden. Dabei gelten keine Einschränkungen auf die konkrete Ausführung des Reservoirs 21 und des Grabens, beziehungsweise der Gräben. Weiterhin gelten keine Einschränkungen hinsichtlich der Anzahl der Gräben und/oder Öffnungen 24 im Reservoir 21, beziehungsweise der Vertiefung 25. Sofern anwendbar können alle beschriebenen Ausführungsformen für die Vertiefung und den Graben, beziehungsweise für das Reservoir 21 und den Kanal 22, im Zusammenhang mit dem Zellkulturträgerbausatz und dem Verfahren Verwendung finden.

## Patentansprüche

1. Zellkulturträger (10), umfassend:
einen ersten Kanal (22); und
ein Reservoir (21),
wobei der erste Kanal (22) und das Reservoir (21) im Inneren des Zellkulturträgers (10) ausgebildet sind,
wobei der erste Kanal (22) eine Einlassöffnung (27) in einer Außenseite des Zellkulturträgers (10) und eine Auslassöffnung (27) in der Außenseite des Zellkulturträgers (10) aufweist;
wobei das Reservoir (21) mindestens zwei nebeneinander angeordnete Öffnungen (24) aufweist;
wobei der erste Kanal (22) durch die mindestens zwei Öffnungen (24) mit dem Reservoir (21) verbunden ist; und
wobei das Reservoir (21) durch keinen weiteren Kanal mit der Außenseite des Zellkulturträgers (10) verbunden ist.

2. Zellkulturträger (10) nach Anspruch 1, wobei das Reservoir (21) eine kreisförmige, elliptische oder rechteckige Querschnittsfläche aufweist, und/oder
wobei ein Verhältnis zweier Hauptausdehnungen des Querschnitts des Reservoirs (21) im Bereich zwischen 0,5 und 2 liegt, und
wobei die Querschnittsfläche parallel zu einer Grundfläche des Zellkulturträgers (10) liegt.

3. Zellkulturträger (10) nach einem der vorangegangenen Ansprüche, umfassend einen zweiten Kanal (22),
wobei der zweite Kanal (22) durch mindestens zwei weitere Öffnungen (24) mit dem Reservoir (21) verbunden ist.

4. Zellkulturträger (10) nach Anspruch 3, wobei die Öffnungen (24) zwischen dem Reservoir (21) und dem ersten Kanal (22) und die Öffnungen zwischen dem Reservoir (21) und dem zweiten Kanal (22) an gegenüberliegenden Stellen im Reservoir (21) angeordnet sind.

5. Zellkulturträger (10) nach einem der vorangegangenen Ansprüche, wobei ein Abstand zwischen zwei der mindestens zwei benachbarten Öffnungen (24) größer als der halbe Wert einer Breite einer der Öffnungen (24) und/oder kleiner als der fünffache Wert der Breite einer der Öffnungen (24) ist.

6. Zellkulturträger (10) nach einem der vorangegangenen Ansprüche, wobei das Reservoir (21) teilweise oder vollständig mit einer zellabweisenden Schicht beschichtet ist.

7. Zellkulturträger (10) nach einem der vorangegangenen Ansprüche, umfassend
ein Deckelement (20), und
ein Bodenelement (30),
wobei auf der dem Bodenelement (30) zugewandten Seite des Deckelements (20) ein Graben und eine Vertiefung (25) ausgebildet sind,
wobei das Deckelement (20) und das Bodenelement (30) flächig miteinander verbunden sind, sodass der Graben und die Vertiefung (25) durch das Bodenelement (30) abgedeckt sind, und
wobei durch die Abdeckung des Grabens der Kanal (22) und durch die Abdeckung der Vertiefung (25) das Reservoir (21) gebildet werden.

8. Zellkulturträger (10) nach Anspruch 7, weiterhin umfassend einen Klebefilm (40), der zwischen dem Deckelement (20) und dem Bodenelement (30) angeordnet ist, sodass das Deckelement (20) und das Bodenelement (30) flächig miteinander verbunden und verklebt sind.

9. Zellkulturträger (10) nach Anspruch 7 oder 8, wobei zwei der mindestens zwei nebeneinander angeordneten Öffnungen (24) des ersten Kanals (22) durch eine Säule (23) getrennt sind,
wobei die Säule (23) im Deckelement (20) ausgebildet ist und das Bodenelement (30) berührt, und
wobei die dem Deckelement (20) zugewandte Seite des Bodenelements (30) plan ausgebildet ist.

10. Zellkulturträger (10) nach einem der vorangegangenen Ansprüche, wobei das Reservoir (21) mit einem Gel (G) befüllt ist, das Zellen oder Zellaggregate enthält.

11. Zellkulturträgerbausatz, umfassend:
ein Deckelement (20) umfassend:
einen Graben; und
eine Vertiefung (25),
wobei die Vertiefung (25) mindestens zwei nebeneinander angeordnete Öffnungen (24) aufweist; und
wobei der Graben durch die mindestens zwei Öffnungen (24) mit der Vertiefung (25) verbunden ist;
einen Klebefilm (40); und
ein Bodenelement (30),
wobei das Bodenelement (30) mittels des Klebefilms (40) derart am Deckelement (20) befestigbar ist, sodass die Vertiefung (25) und der Graben durch das Bodenelement (30) abgedeckt sind, und
wobei ein Zusammenbau des Zellkulturträgerbausatzes einen Zellkulturträger (10) nach einem der vorangegangenen Ansprüche ergibt.

12. Zellkulturträgerbausatz nach Anspruch 11, wobei der Klebefilm (40) auf dem Deckelement (20) aufgebracht ist, sodass die Vertiefung (25) und der Graben nicht mit dem Klebefilm (40) bedeckt sind, und
wobei die dem Deckelement (20) abgewandte Seite des Klebefilms (40) mit einer Schutzfolie (41) bedeckt ist.

13. Verfahren zum Einbringen von Zellen oder Zellaggregaten in einen Zellkulturträger (10), umfassend:
Bereitstellen eines Zellkulturträgerbausatzes nach einem der Ansprüche 11 oder 12;
Befüllen der Vertiefung (25) im Deckelement (20) mit einem Gel (G), das Zellen oder Zellaggregate enthält; und
Abdecken der Vertiefung (25) und des Grabens mit dem Bodenelement (30),
wobei das Deckelement (20) und das Bodenelement (30) flächig miteinander verbunden sind,
wobei das Bodenelement (30) mittels des Klebefilms (40) derart am Deckelement (20) befestigt wird, sodass die Vertiefung (25) und der Graben durch das Bodenelement (30) verschlossen sind; und
wobei das Abdecken der Vertiefung (25) nach dem Befüllen der Vertiefung (25) durchgeführt wird.

14. Verfahren nach Anspruch 13, wobei das Gel (G) mittels 3D-Druck in die Vertiefung (25) eingefüllt wird.

15. Verfahren nach Anspruch 13 oder 14, weiterhin umfassend:
Anschließen der Einlassöffnung (27) und die Auslassöffnung (27) des Kanals (22) an eine Perfusion, sodass der Kanal (22) von einem Zellmedium durchströmt wird.
